(19) Europäisches Patentamt
European Patent Office
Office européen des brevets

(11) Publication number: **0 195 316**
**A1**

# (12) EUROPEAN PATENT APPLICATION

(21) Application number: 86102938.7

(22) Date of filing: 06.03.86

(51) Int. Cl.[4]: C 07 D 401/04
A 61 K 31/47

(30) Priority: 08.03.85 JP 46216/85
04.02.86 JP 22296/86

(43) Date of publication of application:
24.09.86 Bulletin 86/39

(84) Designated Contracting States:
AT BE CH DE FR GB IT LI LU NL SE

(71) Applicant: KYORIN PHARMACEUTICAL CO., LTD.
No. 5, Kanda Surugadai 2-chome
Chiyoda-ku Tokyo(JP)

(72) Inventor: Irikura, Tsutomu
No. 10-28, Ooizumigakuen-cho 7-chome
Nerima-ku Tokyo(JP)

(72) Inventor: Suzue, Seigo
No. 13-4, Aoba 4-chome
Kuki-shi Saitama-ken(JP)

(72) Inventor: Murayama, Satoshi
No. 6095, Tomonuma Nogi-machi
Shimotsuga-gun Tochigi-ken(JP)

(72) Inventor: Hirai, Keiji
No. 1-2-512, Aoba 1-chome
Kuki-shi Saitama-ken(JP)

(72) Inventor: Ishizaki, Takayoshi
No. 1-20-303, Aoba 1-chome
Kuki-shi Saitama-ken(JP)

(74) Representative: Patentanwälte TER MEER - MÜLLER - STEINMEISTER
Mauerkircherstrasse 45
D-8000 München 80(DE)

## (54) Quinolonecarboxylic acid derivatives.

(57) Quinolonecarboxylic acid derivative of the following formula:

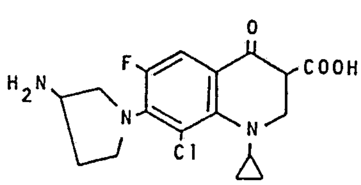

hydrates and pharmaceutically acceptable salts thereof are useful as an antibacterial agent.

EP 0 195 316 A1

Title of the invention:

Quinolonecarboxylic acid derivative and process for its preparation

Detailed description of the invention:

The present invention relates to a novel quinolonecarboxylic acid derivative of the following formula (I),

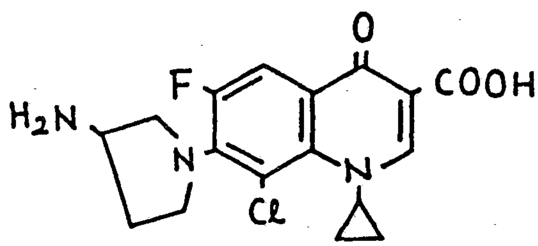

(I)

its hydrates and salts, preparation process therefor and uses thereof as an antibacterial agent.

The compound represented by the formula (I), contains optical isomers, owing to an asymmetric carbon on the aminopyrrolidine ring, the 7-substituent. But all of optical isomers and their mixture are represented, for convenience, by the unitary formula. Hence, the scope of the invention is not limited to one of the

optical isomers or their mixture.

Quinolonecarboxylic acid antibacterial agent began with nalidixic acid and have been developed to piromidic acid, furthermore, to pipemidic acid. They are useful agents for the medical treatment against urinary tract infections with aerobic gram-negative bacteria.

Norfloxacin, which has been developed by us recently, exhibits potent antibacterial activity not only against gram-negative bacteria but also against gram-positive bacteria. Moreover, its potency is extremely stronger than the previous quinolonecarboxylic acids. Norfloxacin became an epoch-making progress in this field, it has been used clinically very often at the present time.

The later quinolonecarboxylic acids, such as, ofloxacin, ciprofloxacin and CI-934, which have substituents similar to norfloxacin, have been continuously developed. CI-934 possesses more potent activity against gram-positive bacteria.

Ciprofloxacin possesses stronger antibacterial activity than that of norfloxacin. But the antibacterial potency against gram-positive bacteria is fairly inferior to that against gram-negative bacteria. The antibacterial activity of CI-934 against gram-positive bacteria is slightly stronger, but that against gram-negative bacteria is lower.

On the other hand, the increase of ß-lactam antibiotics-resistant gram-positive bacteria such as methicillin and cephalosporin resistant *Staphylococcus aureus*, *S. epidermidis*, *Enterococcus faecalis*, have made a trouble in clinical field.

In addition, it has been evidenced that obligate anaerobes on skin or mucous membrane acted as the pathogen of opportunistic infection owing to the popularization of anaerobes inspection as a result of the development of clinical testing technology. It has been reported that anaerobes are found with or without an aerobic bacteria at the rate of 50-80 % on respiratory tract infections, intraperitoneal infections, chronic otitis media, paranasal sinusitis and obstetrical and gynecological infections, and the rate of the combination of anaerobes with Escherichia coli, Enterococcus faecalis and the other Streptococci is about 95 %. Gradually increasing of the resistance rate of anaerobes to ß-lactam antibiotics or clindamycin, which were inherently effective against anaerobes, caused serious problem for choice of chemotherapeutic agents.

Mycoplasma is known as a pathogen of respiratory tract infections, meningitis, endocarditis, arthritis and so on. For infections due to Mycoplasma, tetracyclines or macrolides were usually used, but in recent years, some Mycoplasma becomes highly resistant to these drugs, especially to macrolides. Therefore, in the future, it will be a trouble in the chemotherapy.

Chlamydia, Ureaplasma and Gardneralla vaginalis recently have become a subject of discussion as pathogens of sexual infections. A effective new drug for these bacteria is also required.

On these background, the development of new antimicrobial agents with stronger activity and broader spectrum is required.

We had found norfloxacin which touched off today's prosperity of new quinolone antibacterial agents and had also studied

mode of action. As a result, it was found that the target of quinolones was the DNA gyrase which controls topology of double helix DNA strands.

Moreover, it was recognized that the antibacterial activity of quinolonecarboxylic acids related significantly to the penetrability through bacterial membrane, in particular, to the penetrability through the portion called porins. Based on these knowledges, the concept on new quinolines was established to coincide with the following paragraphs.

1) To act on the DNA gyrase.
2) To have good permeability through the outer membranes, in particular, porins.
3) To be effective surely against gram-positive bacteria, in particular, Staphylococci.
4) Not to lower the activity against Gram-negative bacteria.
5) To act strongly against Serratia sp. and Pseudomonas aeruginosa which show resistance to other antimicrobial agents.
6) To show the activity against anaerobic bacteria.
7) To possess good absorption and not to be metabolized easily.
8) To be excellent in the productivity.
9) To be excellent in the selective toxicity and not to exhibit the harmful side effect for living bodies.

As the compounds possible to satisfy such concept, especially paragraph 9), approximately, we synthesized novel substances represented by the formula (I), and, as a result of the various investigation including its mode of action, the present invention

has been completed.

The special feature of compounds of the investigation in chemical structure is that they have a fluorine atom at 6-position, a chlorine atom at 8-position and a 3-aminopyrrolidinyl group at 7-position in the skeleton of quinolonecarboxylic acid.

Previously, we found norfloxacin introduced a fluorine atom to 6-position in the skeleton of quinolonecarboxylic acid. Since norfloxacin was proved to have high usefulness for the bacterial infections clinically, the antibacterial agents of this class have been studied earnestly at domestic and foreign country, and an epoch-making progress was brought about being called new quinolones or fluoroquinolones.

As a result of the continuation of our tireless study thereafter, it was noticed that the substituent on 8-position also played extremely important roles in the extension of antibacterial spectrum, the increase in activity and the oral absorption as a drug. Namely, various substituents were introduced to 8-position by us, and these compounds were analyzed using techniques such as quantitative structure activity relationship. We have reached to the conclusion that a chlorine atom quite unexpectedly is optimal as the substituent at 8-position.

Finaly, we developed a series of compounds which had a fluorine atom at the 6-position and a chlorine atom at the 8-position as applicated previously. Especially, it was considered that 8-chloro-1-cyclopropyl-6-fluoro-1,4-dihydro-7-(3-methyl-1-piperazinyl)-4-oxo-3-quinolinecarboxylic acid (compound of ref.2) was one of the most excellent compounds among any quinolonecarboxylic acid derivatives because of its broad and strong antibacterial activity.

But unfortunately, it was found that this compound had some disadvantage as the drug used clinically for human. For example, strong vomiting and clonic convulsion was observed in repeated oral administration to dog, suggested that it had side effect on central nervous system. Furthermore, marked elevation of GPT and decrease of TTT at this test seemed to be closely related to hepatoxicity of this compound. The results as mentioned above indicated that it might be impossible to use it for human.

Based on these study, the inventors continued to research the drugs having more potent antibacterial activity and higher safety and then accomplished this invention.

Furtheremore, it was surprising that the compound of invention exhibited far stronger antibacterial activity against anaerobic bacteria, Mycoplasma, Ureaplasma, Chlamydia and Gardnerella vaginalis against which all of the older quinolonecarboxylic acids were low-active or inactive. And the lower mutation frequency of bacteria was an advange of this compound. It was found that, in animals, the compound showed good oral absorption, excellent tissue distribution, satisfying biological stability and acceptability.

In following, explanation is made about the preparation process for the compound of the invention.

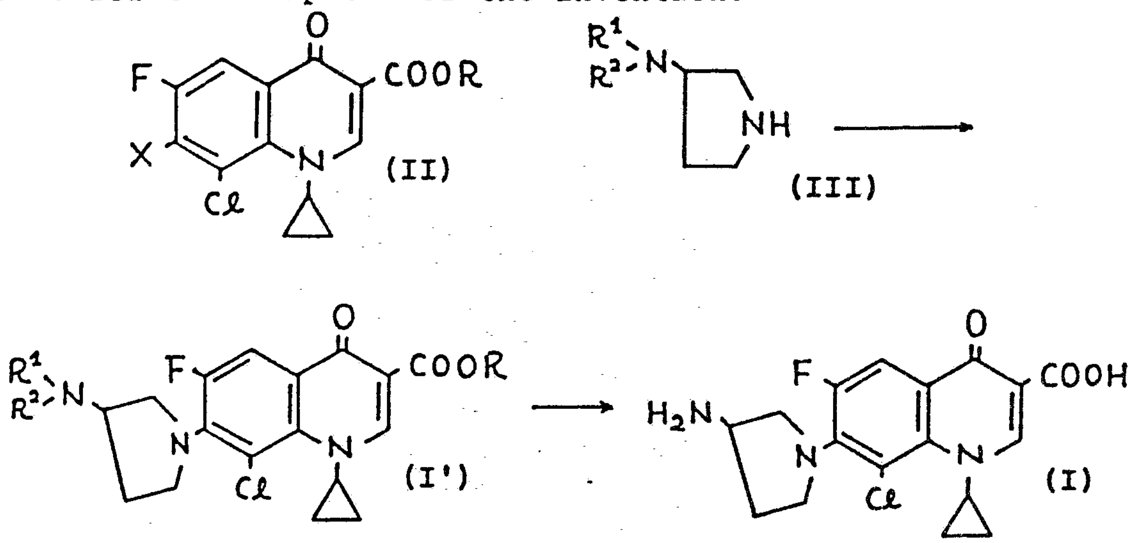

(II) (III)

(I') (I)

wherein R is a hydrogen atom or a lower alkyl group, $R^1$ and $R^2$ are each independently a hydrogen atom or a protecting group of an amino group, or are a protecting group of an amino group which work together and X is a halogen atom.

Namely, by allowing compounds represented by the formula (II) to react with amines represented by the formula (III), compound of the invention represented by the formula (I) is synthesized. However, in the case of compounds wherein both or either of $R^1$ and $R^2$ are protecting group of an amino group in the formula (III), e.g., $R^1$ is a hydrogen atom and $R^2$ is a lower acyl group, for example, an acetyl group, a propionyl group and so on, a lower alkoxycarbonyl group, e.g., a methoxycarbonyl group, an ethoxycarbonyl group, a t-butoxycarbonyl group and so on, or $R^1$ and $R^2$ are a phthaloyl group which work together, the resultants with the compounds represented by the formula (I') are removed the protecting group according to the usual method to give the compounds of the invention. Moreover, in the case of compounds

wherein R is a lower alkyl group in the formula (II), the reaction resultants with the compounds represented by the formula (III) are hydrolyzed according to the usual method and ester is converted to carboxylic acid to offer the compound of the invention. The reaction of compounds represented by the formula (II) with compounds represented by the formula (III) preferably is carried out by heating the mixture in a solvent such as water, alcohols, acetonitrile, dimethylformamide (DMF), dimethyl sulfoxide (DMSO), hexamethylphosphoric triamide, pyridine, picoline and the like or in the absence of the solvent. The reaction temperature is selected appropriately in a range of room temperature to 200 °C, preferablely room temperature to 160 °C. In more details, it is preferable to allow compounds represented by the formula (II) to react with 1 to 5 times mole of compounds represented by the formula (III) for 1 to several hours at room temperature to 160 °C in 2 to 10 times volume of aforementioned solvents. At this time, the use of deacidifying agents such as triethylamine, diazabicyclo bases and potassium carbonate is also desirable. Moreover, compounds (I') wherein R is a lower alkyl group in the formula (I) can be hydrolyzed according to the usual method. Such hydrolysis can be carried out easily with alkalies such as potassium hydoxide or acids such as sulfuric acid at room temperature to boiling point of solvents in water, mixed liquor of water with alcohols, mixed liquor of water with acetic acid, and so on.

The starting compounds of the formula (II) are also new and obtained by the following procedures.

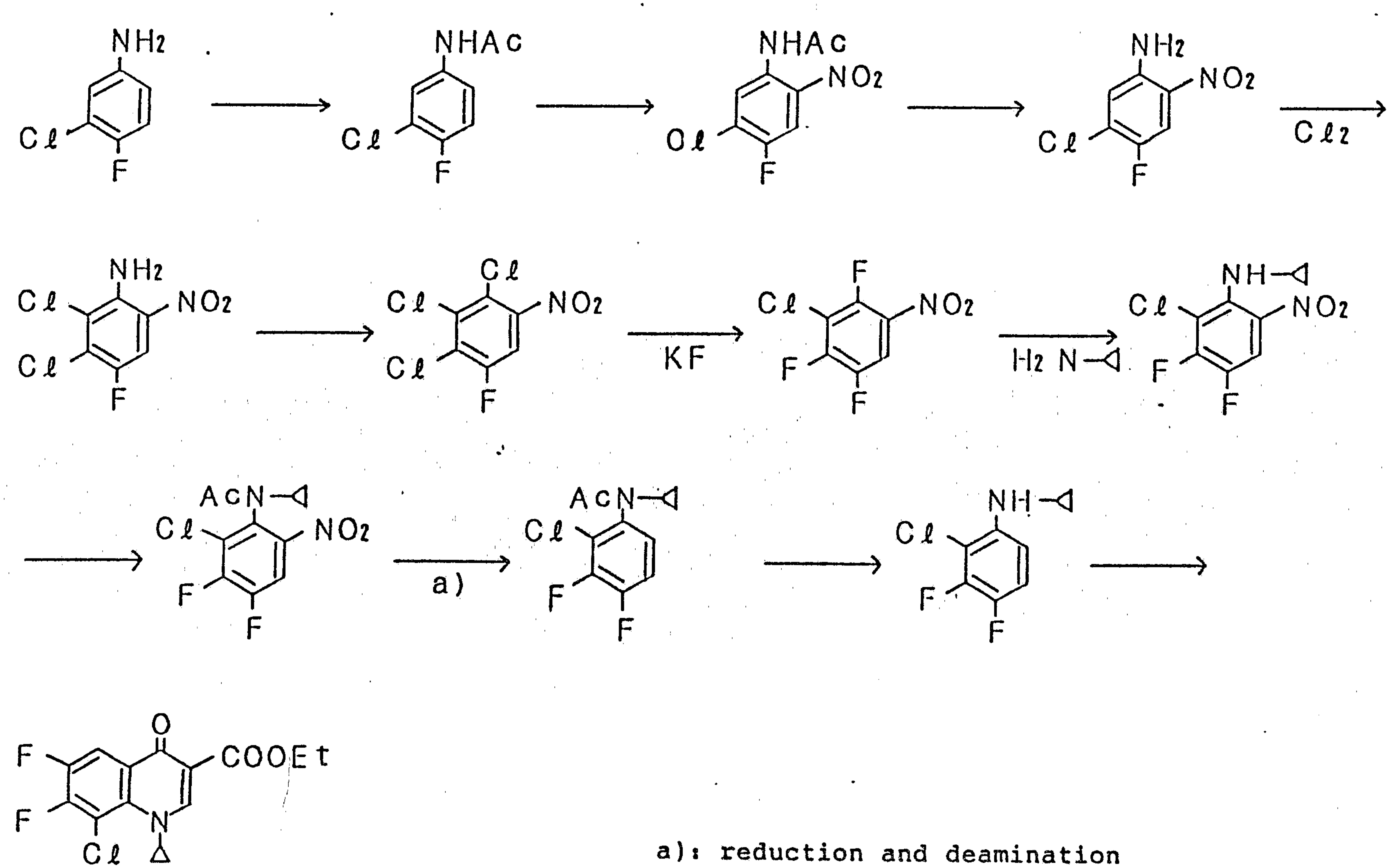


a): reduction and deamination

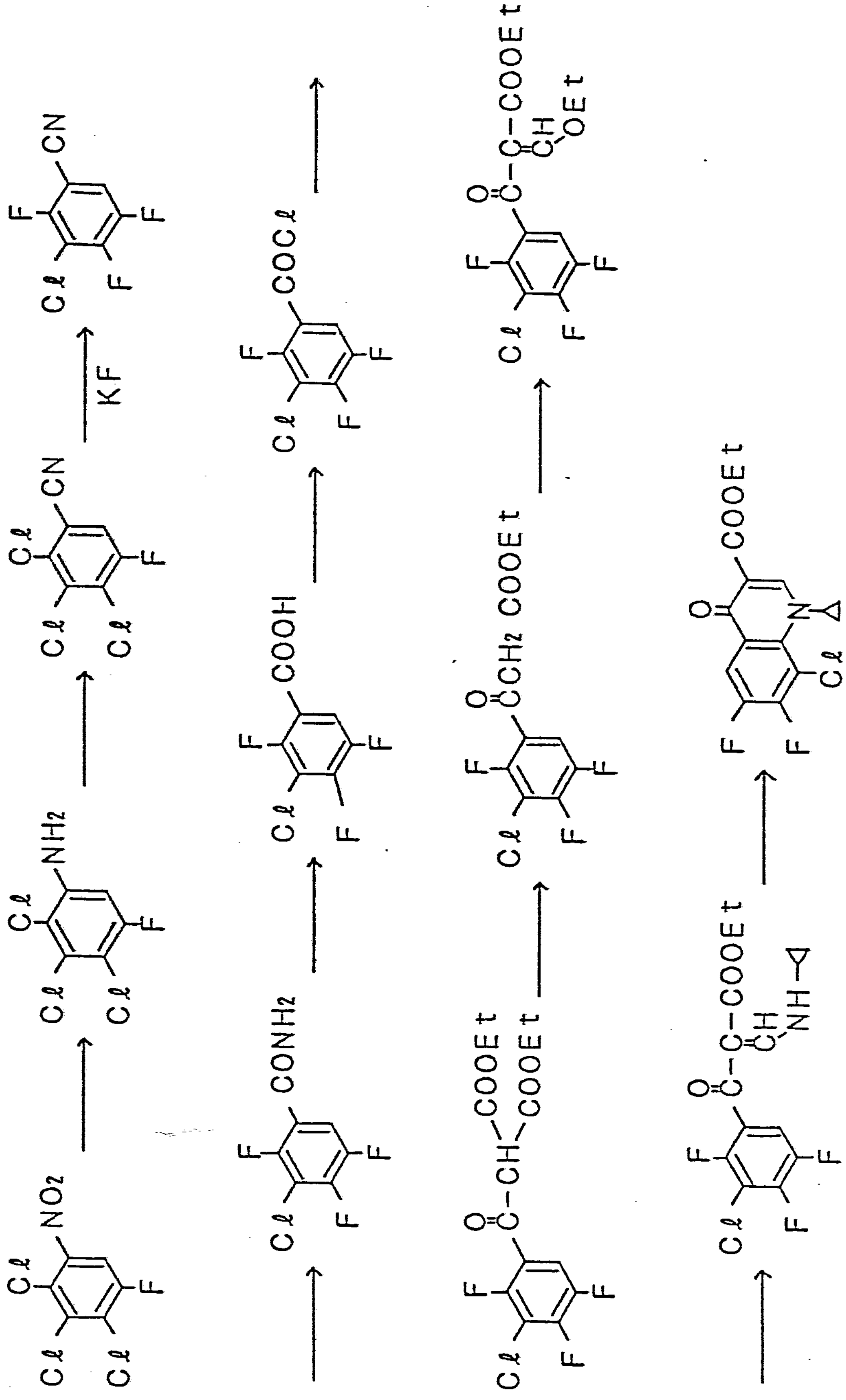
KF

Furthermore, the compounds of the formula (I) can be converted, if desired, to the pharmaceutically acceptable ammonium salts or carboxylic acid metal salts by treatment with acid or alkali. The acid may be organic or inorganic acids such as, for example, hydrochloric acid, sulfuric acid, phosphoric acid, acetic acid, methanesulfonic acid, oxalic acid and lactic acid. The carboxylic acid metal salts may be, for example, sodium, potassium, magnesium, calcium, aluminum, cerium, chromium, cobalt, copper, iron, zinc, platinum and silver salts.

The compound of the formula (I), hydrates and salts thereof may be used as medicines in the conventional form of pharmaceutical preparations, which may be, for example, tablets, capsules, powder, ointments, suppositories, injections or eye drops, suitable for peroral, parenteral, enteral or local administration.

The following examples will further illustrate the present invention without, however, limiting it thereto.

Example 1 N-(3-Chloro-4-fluorophenyl)acetamide

To 3-chloro-4-fluoroaniline (100 g) was slowly added acetic anhydride (200 ml). After allowed to stand for 30 minutes, the reaction mixture was poured into water (1 litter). The resulting precipitate was collected by filtration and recrystallized from aqueous ethanol to give the title compound (119.4 g), mp 118-119 °C.

Example 2 N-(3-Chloro-4-fluoro-6-nitrophenyl)acetamide

To a solution of N-(3-chloro-4-fluorophenyl)acetamide (55 g) in concentrated sulfuric acid (165 ml) was added dropwise concentrated nitric acid (d 1.42, 154 ml) at 5-10 °C during an hour

with stirring in an ice-salt bath. After stirring for an hour at the same temperature, the reaction mixture was poured into ice water. The resulting precipitate was collected by filtration, sufficiently washed with water and recrystallized from acetonitrile to give the title compound (48.9 g) as yellow needles, mp 114-115 °C.

Analysis (%) for $C_8H_6ClFN_2O_3$, Calcd. (Found): C, 41.31 (41.48); H, 2.60 (2.52); N, 12.04 (12.13).

Example 3 3-Chloro-4-fluoro-6-nitroaniline

A solution of N-(3-chloro-4-fluoro-6-nitrophenyl)acetamide (30 g) in concentrated hydrochloric acid (50 ml) and ethanol (200 ml) was refluxed for 2.5 hours. To the reaction mixture was added ice water (300 ml) and the resulting precipitate was collected by filtration, washed with water and dried to give the title compound (24.9 g) as yellow needles, mp 149.5-150 °C.

Analysis (%) for $C_6H_4ClFN_2O_2$, Calcd. (Found): C, 37.82 (37.85); H, 2.11 (2.03); N, 14.70 (14.80).

Example 4 2,3-Dichloro-4-fluoro-6-nitroaniline

Into a solution of 3-chloro-4-fluoro-6-nitroaniline (14.3 g) in acetic acid (150 ml) was boubled chlorine gas at 18-20 °C during 70 minutes. The reaction mixture was poured into ice water (300 ml) and the resulting precipitate was collected by filtration, washed with water and recrystallized from ethanol to give the title compound (14.33 g) as yellow needles, mp 161 °C.

Analysis (%) for $C_6H_3Cl_2FN_2O_2$, Calcd. (Found): C, 32.03 (32.17); H, 1.34 (1.26); N, 12.45 (12.65).

Example 5 2,3,4-Trichloro-5-fluoronitrobenzene

To a mixture of anhydrous cupric chloride (13.58 g) and t-butylnitrite (12.4 g) in anhydrous acetonitrile (100 ml) was added portionwise 2,3-dichloro-4-fluoro-6-nitroaniline (18.05 g) at 60-62 °C during 30 minutes. After stirring for 30 minutes at 60-65 °C, the reaction mixture was poured into chilled 10% diluted hydrochloric acid (300 ml) and extracted with benzene. The organic layer was successively washed with diluted hydrochloric acid and water, dried over anhydrous sodium sulfate and concentrated. The resulting residue was purified by distillation to give the title compound (17.26 g), bp 137-142 °C/27 mmHg.

NMR (δ in $CDCl_3$), 7.65 (d, J=7.5 Hz)

Example 6 3-Chloro-2,4,5-trifluoronitrobenzene

To a suspension of potassium fluoride (64.9 g) in anhydrous dimethyl sulfoxide (230 ml) was added 2,3,4-trichloro-5-fluoronitrobenzene (54.4 g) at 140 °C and stirred at the same temperature for 10 minutes. The reaction mixture was poured into ice water (700 ml) and extracted with petroleum ether. The organic layer was successively washed with water, aqueous potassium carbonate solution and then water, dried over anhydrous sodium sulfate and concentrated. The resulting residue was distilled to give the title compound (9.7 g), bp 95-108 °C/30mmHg.

NMR (δ in $CDCl_3$), 7.94 (ddd, J=6.7, 7.6, 9.0 Hz)

Example 7 3-Chloro-2-cyclopropylamino-4,5-difluoronitrobenzene

A solution of cyclopropylamine (2.8 g) and triethylamine (5.1 g) in anhydrous toluene (20 ml) was added dropwise to a

solution of 3-chloro-2,4,5-trifluoronitrobenzene (9.7 g) in anhydrous toluene (30 ml) at 3-5 °C during 40 minutes with stirring. After stirring for 3 hours at the same temperature, the reaction mixture was poured into ice water (150 ml) and extracted with dichloromethane. The organic layer was washed with water, dried over anhydrous sodium sulfate and then concentrated. The resulting residue was purified by silica gel chromatography using n-hexane-dichloromethane as an eluent to give the title compound (4.4 g) as red oil.

NMR (δ in $CDCl_3$), 0.5-1.0 (4H, m, 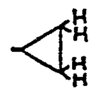), 3.0-3.2 (1H, m, 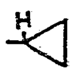), 7.19 (1H, s, NH), 7.85 (1H, dd, J=8.2, 9.9 Hz, 5-H).

Example 8 N-(2-Chloro-3,4-difluoro-6-nitrophenyl)-N-cyclopropylacetamide

To 3-chloro-2-cyclopropylamino-4,5-difluoronitrobenzene (4.4 g) was added acetic anhydride (15 ml) with one portion and the mixture was stirred for 30 minutes at room temperature, and then poured into ice water (100 ml). Excessive acetic anhydride was decomposed by potassium carbonate powder, and then the mixture was allowed to stand overnight at 5 °C. The resulting precipitate was collected by filtration and recrystallized from ethyl acetate-n-hexane to give the title compound (2.7 g), mp 98-99.5 °C.

Analysis (%) for $C_{11}H_9ClF_2N_2O_3$, Calcd. (Found): C, 45.46 (45.56); H, 3.12 (3.00); N, 9.64 (9.69).

Example 9 N-(2-Chloro-3,4-difluorophenyl)-N-cyclopropylacetamide

A mixture of N-(2-chloro-3,4-difluoro-6-nitrophenyl)-N-

cyclopropylacetamide (2.7 g) and 10 % palladium-charcoal (0.5 g) in ethanol (50 ml) was hydrogenated for 40 minutes at 2-3 °C under atmospheric pressure in an ice bath. The catalyst was filtered off and the filtrate was concentrated. Then, the resulting crystalline residue was dried in vacuo at room temperature for 10 hours. A solution of the residue in anhydrous dimethylformamide (15 ml) was added dropwise to a solution of t-butyl nitrite (1.72 g) in anhydrous dimethylformamide (10 ml) at 50-52 °C during 13 minutes. The reaction mixture was stirred at the same temperature for 5 minutes, then poured into ice water and extracted with ether. The organic layer was successively washed with water, dilute hydrochloric acid and water, dried over anhydrous sodium sulfate and concentrated. The resulting residue was purified by silica gel chromatography using n-hexane-ethyl acetate as an eluent and recrystallized from petroleum ether to give the title compound (0.44 g), mp 60.5-61.5 °C.

Analysis (%) for $C_{11}H_{10}ClF_2NO$, Calcd. (Found): C, 53.78 (53.87); H, 4.10 (4.02); N, 5.70 (5.78).

Example 10 N-Cyclopropyl-2-chloro-3,4-difluoroaniline

A solution of N-(2-chloro-3,4-difluorophenyl)-N-cyclopropylacetamide (0.44 g) in 20 % diluted hydrochloric acid (7 ml) was heated at 80-100°C for 6 hours with stirring, and then cooled. The reaction mixture was poured into ice water, alkalized with aqueous sodium hydroxide and extracted with ether. The ether layer was washed with water, dried over anhydrous sodium sulfate and then concentrated. The resulting residue was purified by silica gel chromatography using petroleum ether as an eluent to

give the title compound (100 mg) as orange oil.

Example 11 Ethyl 8-chloro-1-cyclopropyl-6,7-difluoro-1,4-dihydro-4-oxo-3-quinolinecarboxylate

A mixture of N-cyclopropyl-2-chloro-3,4-difluoroaniline (100 mg) and diethyl ethoxymethylenemalonate (100 mg) was stirred for 10.5 hours at 100-135 °C with removal of generated ethanol by flowing nitrogen gas, and then cooled. Polyphosphoric acid (1 g) was mixed with this, and the mixture was stirred for 3 hours at 125-135 °C. After cooling, the reaction mixture poured into ice water, extracted with chloroform. The organic layer was successively washed with aqueous potassium carbonate and water, dried over anhydrous sodium sulfate and then concentrated. The resulting residue was purified with silica gel thin layer chromatography using ether as an eluent to give the title compound (11 mg) as colorless needles, mp 160-162.5 °C.

NMR (δ in $CDCl_3$), 1.0-1.5 (4H, m, 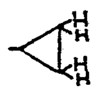), 1.40 (3H, t, J=7.0 Hz, $-\underline{CH_3}$), 4.1-4.4 (1H, m, 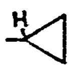), 4.38 (2H, q, J=7.0 Hz, $-\underline{CH_2}-CH_3$), 8.22 (1H, dd, J=8.8, 9.7 Hz, 5-H), 8.66 (1H, s, 2-H)

Example 12 2,3,4-Trichloro-5-fluoroaniline

To a suspension of iron powder (54.6 g) in water (60 ml), with vigorous stirring at 50-60 °C, was slowly added concentrated hydrochloric acid (6.7 ml). After ethanol (150 ml) was mixed, 2,3,4-trichloro-5-fluoronitrobenzene (75.1 g) was added portionwise to the suspension at 60-70 °C during an hour. After stirring for an hour at 80 °C, the hot reaction mixture was filtered and the insoluble material was successively washed with hot ethanol (100 ml) and benzene (300 ml). The filtrate and washings

were combined and mixed with ice water. The resulting organic layer was collected and the water layer was extracted with benzene (200 ml). The organic layers were washed with water, dried over anhydrous sodium sulfate and then concentrated. The resulting residue was recrystallized from n-hexane to give the title compound (58.6 g) as light brown needles, mp 118-120 °C.

Example 13 2,3,4-Trichloro-5-fluorobenzonitrile

To a suspension of 2,3,4-trichloro-5-fluoroaniline (43.8 g) in concentrated hydrochloric acid (300 ml) with vigorous stirring was added sodium nitrite (21.1 g) in water (50 ml) at -2∿0 °C for 20 minutes. After stirred for 30 minutes, the mixture was poured into ice water (300 ml) containing sodium tetrafluoroborate (67.2 g), stirred vigorously and then allowed to stand for 30 minutes in an ice bath. The resulting precipitate was collected by filtration and successively washed with chilled water and ether. This faint yellow precipitate was added portionwise during 30 minutes to a solution of cuprous cyanide (36.5 g), potassium cyanide (53.0 g) and sodium carbonate (11.1 g) in water (300 ml) with vigorous stirring at room temperature. After the mixture was stirred for 30 minutes, benzene (300 ml) was added to the suspension and then the mixture was stirred for 15 minutes. The insoluble material was collected by filtration, and washed with benzene (150 ml). The filtrate and washings were combined and successively washed with 20% aqueous potassium cyanide and water, dried over anhydrous sodium sulfate and then concentrated. The resulting residue was recrystallized from n-hexane to give the title compound (27 g) as light brown needles, mp 97-99 °C.

Example 14 3-Chloro-2,4,5-trifluorobenzonitrile

To a solution of potassium fluoride (31.7 g) in dimethyl sulfoxide (100 ml) with stirring at 130°C was added 2,3,4-trichloro-5-fluorobenzonitrile (15 g) and then the mixture was stirred for 1.5 hours at 140 °C. After cooling, the reaction mixture was poured into ice water (300 ml) and extracted with dichloromethane. The organic layer was washed with water, dried over anhydrous sodium sulfate and concentrated to give the title compound (11.9 g) as pale brown oil.

Example 15 3-Chloro-2,4,5-trifluorobenzamide

A solution of 3-chloro-2,4,5-trifluorobenzonitrile (11.9 g) in 30 % hydrogen bromide-acetic acid (150 ml) was refluxed for 80 minutes, poured into ice water (350 ml) and extracted with ether. The ether layer was successively washed with 1 N-potassium hydroxide solution and water, dried over anhydrous sodium sulfate and concentrated. The residue was purified by silica gel chromatography eluting with n-hexane-ethyl acetate to give the title compound (3.97 g), mp 110-113.5 °C.

Example 16 3-Chloro-2,4,5-trifluorobenzoic acid

A mixture of 3-chloro-2,4,5-trifluorobenzamide (3.97 g) and 18 N-sulfuric acid (20 ml) was stirred at 125-135 °C for 9 hours, and then poured into ice water (100 ml). After standing overnight, the resulting precipitate was collected by filtration. The mother liquor was extracted with ether, and the ether layer was dried over anhydrous sodium sulfate, then concentrated and mixed to the precipitate. A solution of the above precipitate and residue in dichloromethane (150 ml) was filtered through a

celite pad and the filtrate was concentrated to give the title compound (2.38 g), mp 115-116.5 °C.

Analysis (%) for $C_7H_2ClF_3O_2$, Calcd. (Found): C, 39.93 (40.18); H, 0.96 (0.80).

Example 17 3-Chloro-2,4,5-trifluorobenzoyl chloride

A solution of the 3-chloro-2,4,5-trifluorobenzoic acid (2.38 g) in thionyl chloride (10 ml) was refluxed for 2.5 hours, and then concentrated. The resulting residue was purified by distillation in nitrogen atomosphere to give the title compound (1.99 g), bp 88 °C/19 mmHg.

Example 18 Diethyl 3-chloro-2,4,5-trifluorobenzoylmalonate

Magnesium turnings (0.22 g) and carbon tetrachloride (0.1 ml) was added to absolute ethanol (1.5 ml). To the stirring suspension was added dropwise a solution of diethyl malonate (1.4 g) and absolute ethanol (2 ml) in toluene (6 ml) during 28 minutes at 47-60 °C. The mixture was stirred for 80 minutes, and then cooled in an acetone-dry ice bath. A solution of 3-chloro-2,4,5-trifluorobenzoyl chloride (1.99 g) in anhydrous toluene (2 ml) was added dropwise to the resulting solution at -12 ∿ -8 °C during 13 minutes. The mixture was stiired for 2 hours at -10 ∿ -5 °C allowed to stand overnight at room temperature, and then mixed with ice water (6 ml) containing concentrated sulfuric acid (0.4 ml). The resulting organic layer was collected and the water layer was extracted with toluene. The combined organic layer was washed with water, dried over anhydrous sodium sulfate and then concentrated to give the title compound (3.05 g) as pale yellow oil.

Example 19 Ethyl 3-chloro-2,4,5-trifluorobenzoylacetate

To an emulsion of diethyl 3-chloro-2,4,5-trifluorobenzoylmalonate (3.05 g) in water (4 ml) was added p-toluenesulfonic acid (4 mg) and refluxed for 4 hours with vigorous stirring. After cooling, the reaction mixture was extracted with dichloromethane. The organic layer was washed with water, dried over anhydrous sodium sulfate and concentrated. The residue was recrystallized from ether-n-hexane to give the title compound (1.22 g), mp 80-83 °C.

Analysis (%) for $C_{11}H_8ClF_3O_3$, Calcd. (Found): C, 47.08 (46.96); H, 2.87 (2.77).

Example 20 Ethyl 2-(3-chloro-2,4,5-trifluorobenzoyl)-3-ethoxyacrylate

A mixture of ethyl 3-chloro-2,4,5-trifluorobenzoylacetate (1.22 g), ethyl orthoformate (0.97 g) and acetic anhydride (1.12 g) was stirred at 118-143 °C for 3 hours and then concentrated to give the title compound (1.4 g) as yellow oil.

Example 21 Ethyl 2-(3-chloro-2,4,5-trifluorobenzoyl)-3-cyclopropylaminoacrylate

To a solution of ethyl 2-(3-chloro-2,4,5-trifluorobenzoyl)-3-ethoxyacrylate (1.4 g) in absolute ethanol (3 ml) was added a solution of cyclopropylamine (0.26 g) in absolute ethanol (2 ml) at 5-10 °C during 15 minutes. The mixture was allowed to stand at 5 °C for 1.5 hours and then stirred at room temperature for an hour. The resulting precipitate was collected by filtration. The filtrate was concentrated, mixed with the precipitate and then recrystallized from petroleum ether to give the title compound (1.09 g), mp 84-85.5 °C.

Analysis (%) for $C_{15}H_{13}ClF_3NO_3$, Calcd. (Found): C, 51.81 (51.76); H, 3.77 (3.74); N, 4.03 (4.03).

Example 22 Ethyl 8-chloro-1-cyclopropyl-6,7-difluoro-1,4-dihydro-4-oxo-3-quinolinecarboxylate

To a solution of ethyl 2-(3-chloro-2,4,5-trifluorobenzoyl)-3-cyclopropylaminoacrylate (1.09 g) in anhydrous dimethylformamide (5 ml) was added sodium fluoride (0.21 g). The mixture was stirred at 130-156 °C for 3.5 hours, and then poured into ice water (50 ml) and the resulting precipitate was collcted by filtration, washed with water and recrystallized from ethyl acetate to give the title compound (0.96 g), mp 158-159 °C.

Analysis (%) for $C_{15}H_{12}ClF_2NO_3$, Calcd. (Found): C, 54.98 (54.96); H, 3.69 (3.57); N, 4.27 (4.25).

Example 23 8-Chloro-1-cyclopropyl-6,7-difluoro-1,4-dihydro-4-oxo-3-quinolinecarboxylic acid

A mixture of ethyl 8-chloro-1-cyclopropyl-6,7-difluoro-1,4-dihydro-4-oxo-3-quinolinecarboxylate (0.24 g), acetic acid (2 ml), water (1.5 ml) and concentrated sulfuric acid (0.25 ml) was refluxed for an hour, and then poured into ice water. The resulting precipitate was collected by filtration and successively washed with water and ether to give the title compound (0.17 g), mp 194-195 °C.

Analysis (%) for $C_{13}H_8ClF_2NO_3$, Calcd. (Found): C, 52.11 (52.00); H, 2.69 (2.53); N, 4.67 (4.64).

Example 24 7-[3-(t-Butoxycarbonylamino)-1-pyrrolidinyl]-8-chloro-1-cyclopropyl-6-fluoro-1,4-dihydro-4-oxo-3-quinolonecarboxylic acid

A mixture of 8-chloro-1-cyclopropyl-6,7-difluoro-1,4-dihydro-4-oxo-3-quinolinecarboxylic acid (500 mg, 1.67 mmol), anhydrous acetonitrile (5 ml), 1,8-diazabicyclo[5,4,0]-7-undecene (DBU; 250 mg, 1.67 mmol) and 3-(t-butoxycarbonylamino)pyrrolidine (430 mg) was refluxed for an hour. The reacting mixture was concentrated under reduced pressure, to the resulting residue was added ethanol-ether and stood for 2 days in a refrigerator. The resulting precipitate was filtered and washed with ethanol-ether (1:1) and ether successively to give the title compound (430 mg, 55.3 %) as pale yellow powder.

$IR\nu^{KBr}_{max}cm^{-1}$ : 3300 (-NH-CO), 1710 ($\overset{OC(CH_3)_3}{/}$C=O, $\overset{OH}{/}$C=O), 1620 (C=O)

NMR ($\delta$ in $CDCl_3$) : 0.80-1.30 (4H, m), 1.45 (9H, s), 1.60-2.50 (3H, m), 3.10-3.96 (4H, m), 4.00-4.60 (1H, m), 7.94 (1H, d, J=13.18 Hz), 8.86 (1H, s).

Example 25 7-(3-Amino-1-pyrrolidinyl)-8-chloro-1-cyclopropyl-6-fluoro-1,4-dihydro-4-oxo-3-quinolinecarboxylic acid

7-[3-(t-Butoxycarbonylamino)-1-pyrrolidinyl]-8-chloro-1-cyclopropyl-6-fluoro-1,4-dihydro-4-oxo-3-quinolinecarboxylic acid (430 mg) was dissolved in a mixture of methanol (5 ml) and concentrated hydrochloric acid (5 ml) and stirred at room temperature for 30 minutes. To the reacting mixture was added concentrated aqueous ammonia to adjust pH to 7, and the resulting precipitate was filtered, washed with water and methanol successively and recrystallized from a mixture of chloroform-methanol-

concentrated aqueous ammonia to give the title compound (110 mg) as milk-white powder, mp 253-258 °C (decompd.).

Analysis (%) for $C_{17}H_{17}ClFN_3O_3$, Calcd. (Found): C, 55.82 (56.09); H, 4.68 (4.82); N, 11.49 (11.46).

Example 26 7-(3-acetamido-1-pyrrolidinyl)-8-chloro-1-cyclopropyl-6-fluoro-1,4-dihydro-4-oxo-3-quinolinecarboxylic acid

A mixture of 8-chloro-1-cyclopropyl-6,7-difluoro-1,4-dihydro-4-oxo-3-quinolinecarboxylic acid (1 g), anhydrous acetonitrile (10 ml), 3-acetamidopyrrolidine (0.64 g) and DBU (0.51 g) was refluxed for an hour. The reacting mixture was concentrated under reduced pressure, the resulting residue was dissolved in chloroform (20 ml) and washed with 10 % aqueous citric acid solution (10 ml). The chloroform layer was further washed with aqueous saturated sodium chloride solution, dried over anhydrous sodium sulfate and concentrated under reduced pressure. The resulting residue was recrystallized from methanol to give the title compound (1.2 g) as pale yellow prisms, mp 210-212 °C.

Analysis (%) for $C_{19}H_{19}ClFN_3O_4 \cdot 1/4\ H_2O$, Calcd. (Found): C, 55.35 (55.39); H, 4.77 (4.68); N, 10.19 (10.12).

Example 27 7-(3-Amino-1-pyrrolidinyl)-8-chloro-1-cyclopropyl-6-fluoro-1,4-dihydro-4-oxo-3-quinolinecarboxylic acid

A mixture of 7-(acetamido-1-pyrrolidinyl)-8-chloro-1-cyclopropyl-6-fluoro-1,4-dihydro-4-oxo-3-quinolinecarboxylic acid (0.8 g) and water (20 ml) containing sodium hydroxide (0.8 g) was refluxed for 5 hours. The reacting mixture was neutralized with

acetic acid, the resulting precipitate was collected by filtration. This precipitate was washed with water, dried and recrystallized from a mixture of methanol-chloroform-concentrated aqueous ammonia to give the title compound (0.41 g) as white plate, mp 238-240 °C (decompd.).

Analysis (%) for $C_{17}H_{17}ClFN_3O_3 \cdot 1/2\ H_2O$, Calcd. (Found): C, 54.48 (54.44); H, 4.84 (4.78); N, 11.21 (11.20).

Example 28 7-(3-Amino-1-pyrrolidinyl)-8-chloro-1-cyclopropyl-6-fluoro-1,4-dihydro-4-oxo-3-quinolinecarboxylic acid

A mixture of 8-chloro-1-cyclopropyl-6,7-difluoro-1,4-dihydro-4-oxo-3-quinolinecarboxylic acid (0.6 g), anhydrous acetonitrile (6 ml), 3-aminopyrrolidine (0.35 g) and DBU (0.31 g) was refluxed for an hour. Then, 3-aminopyrrolidine (0.2 g) was more added and further refluxed for 2 hours. After cooling, the resulting precipitate was collected by filtration, dissolved in water (9 ml) containing sodium hydroxide (0.12 g) and neutralized with acetic acid. The resulting precipitate was collected by filtration and washed with water and acetonitrile successively to give the title compound (0.52 g) as colorless powder, mp 237-238 °C (decompd.).

Analysis (%) for $C_{17}H_{17}ClFN_3O_3 \cdot H_2O$, Calcd. (Found): C, 53.20 (52.97); H, 4.99 (4.62); N, 10.95 (10.83).

Example 29 7-(3-Amino-1-pyrrolidinyl)-8-chloro-1-cyclopropyl-6-fluoro-1,4-dihydro-4-oxo-3-quinolinecarboxylic acid hydrochloride

To a suspension of 7-(3-amino-1-pyrrolidinyl)-8-chloro-1-cyclopropyl-6-fluoro-1,4-dihydro-4-oxo-3-quinolinecarboxylic acid (100 mg) in ethanol (2 ml) was added 0.2 ml of ethanol solution of hydrogen chloride (7.0 mmol HCl/ml) and then the mixture was concentrated. The resulting residue was recrystallized from methanol to give the title compound (79 mg) as light yellow prisms, mp 263-265 °C (decompd.).

Analysis (%) for $C_{17}H_{17}ClFN_3O_3 \cdot HCl$, Calcd. (Found): C, 50.76 (50.50); H, 4.51 (4.44); N, 10.45 (10.38).

Experiment 1. Antibacterial spectrum

Minimal inhibitory concentrations (MICs) were determined in accordance with the method recommended by Japan Society of Chemotherapy. The results are shown in Table 1 and 2. The present compound showed a much stronger antibacterial activity against anaerobic bacteria and aerobic gram-positive bacteria, which were standard or clinical isolated strains, than the reference compounds. Especially, the present compound showed excellent activity against Pseudomonas aeruginosa than the reference compounds.

Table 1-1 In vitro antibacterial activity (standard strains)

| Organism ($10^6$ cells/ml) | Gram | MIC (μg/ml) | | | | |
|---|---|---|---|---|---|---|
| | | Exp.25 | Ref.1 | Ref.2 | CFLX | CI-934 |
| Bacillus subtilis PCI 219 | + | 0.025 | 0.025 | 0.025 | 0.05 | 0.05 |
| Staphylococcus aureus 209 P | + | 0.05 | 0.20 | 0.10 | 0.39 | 0.10 |
| S. aureus IID 670 (Terajima) | + | 0.05 | 0.20 | 0.10 | 0.39 | 0.10 |
| S. epidermidis IID 866 | + | 0.05 | 0.20 | 0.10 | 0.39 | 0.10 |
| Streptococcus pyogenes (S-8) | + | 0.05 | 0.39 | 0.39 | 0.39 | 0.20 |
| S. pyogenes IID 692 | + | 0.10 | 0.78 | 0.78 | 0.78 | 0.39 |
| S. pneumoniae IID 552 | + | 0.10 | 0.78 | 0.39 | 1.56 | 0.39 |
| E. faecalis IID 682 | + | 0.10 | 0.78 | 0.39 | 0.78 | 0.39 |
| Escherichia coli NIHJ JC-2 | - | ≤0.006 | ≤0.006 | ≤0.006 | ≤0.006 | 0.10 |
| E. coli ATCC 10536 | - | 0.0125 | 0.0125 | 0.0125 | 0.0125 | 0.10 |
| Proteus vulgaris IFO 3167 | - | 0.0125 | ≤0.006 | 0.0125 | ≤0.006 | 0.05 |
| P. mirabilis IID 994 | - | 0.0125 | ≤0.006 | 0.0125 | 0.0125 | 0.20 |
| P. morganii IID 602 | - | 0.05 | 0.025 | 0.05 | 0.05 | 0.39 |
| Klebsiella pneumoniae KY(GN)6445 | - | 0.0125 | 0.0125 | 0.025 | 0.0125 | 0.20 |
| K. pneumoniae 1-220S | - | 0.05 | 0.05 | 0.05 | 0.05 | 0.39 |
| Enterobacter cloacae IID 977 | - | 0.05 | 0.025 | 0.05 | 0.05 | 0.78 |
| Citrobacter freundii IID 976 | - | 0.0125 | 0.0125 | 0.05 | ≤0.006 | 0.20 |
| Serratia marcescens IID 618 | - | 0.05 | 0.10 | 0.05 | 0.39 | 0.39 |
| Shigella sonnei IID 969 | - | 0.0125 | ≤0.006 | 0.0125 | ≤0.006 | 0.10 |
| Salmonella enteritidis IID 604 | - | 0.0125 | 0.0125 | 0.05 | 0.025 | 0.39 |
| Pseudomonas aeruginosa V-1 | - | 0.20 | 0.20 | 0.20 | 0.20 | - |
| P. aeruginosa IFO 12689 | - | 0.20 | 0.39 | 1.56 | 0.39 | 6.25 |
| Yersinia enterocolitica IID 981 | - | 0.025 | 0.025 | 0.05 | 0.05 | 0.39 |
| Acinetobacter anitratus IID 876 | - | 0.05 | 0.0125 | 0.05 | 0.025 | 0.39 |
| Alcaligenes faecalis 0104002 | - | 0.05 | 0.025 | 0.20 | 0.39 | 6.25 |

Table 1-2 In vitro antibacterial activity (standard strains)

| Organism ($10^6$ cells/ml) | Gram | MIC (μg/ml) | | | | |
|---|---|---|---|---|---|---|
| | | Exp.25 | Ref.1 | Ref.2 | CFLX | CI-934 |
| Bacteroides fragilis GM 7000 | - | 0.10 | 1.56 | 0.39 | 3.13 | 6.25 |
| B. fragilis 0558 | - | 0.10 | 0.78 | 0.39 | 3.13 | 3.13 |
| B. fragilis 25285 | - | 0.10 | 0.78 | 0.39 | 6.25 | 3.13 |
| B. fragilis 8503 | - | 0.39 | - | 0.78 | 6.25 | 6.25 |
| B. thetaiotaomicron (0661) | - | 0.20 | 3.13 | 0.78 | 25 | 25 |
| Fusobacterium necrophorum S-45 | - | 0.20 | - | 0.39 | 1.56 | - |
| F. varium KYA 8501 | - | 0.78 | 3.13 | 3.13 | 12.5 | 6.25 |
| Eubacterium lentum GAI 5242 | + | 0.10 | - | 0.20 | 0.78 | - |
| Propionibacterium acens 11828 | + | 1.56 | - | 3.13 | 12.5 | - |
| Peptococcus magnus KY 017 | + | 0.05 | - | 0.20 | 0.39 | - |
| Clostridium difficile I-E | + | - | - | - | - | 3.13 |
| C. perfringens KYA 13123 | + | 0.10 | 0.39 | 0.39 | 0.39 | - |
| C. ramosum | + | 0.78 | - | 3.13 | 6.25 | - |
| Peptostreptococcus anaerobius KYA 27337 | + | 0.20 | - | 0.78 | 1.56 | - |
| Pst. microns UPI 5464-1 | + | 0.05 | - | 0.20 | 0.20 | - |
| Veillonella parvula KYA 10790 | - | 0.05 | - | 0.20 | 0.20 | - |

Ref.1 : 1-Cyclopropyl-6,8-difluoro-1,4-dihydro-4-oxo-7-(1-piperazinyl)-3-quinolinecarboxylic acid

Ref.2 : 8-Chloro-1-cyclopropyl-6-fluoro-1,4-dihydro-7-(3-methyl-1-piperazinyl)-4-oxo-3-quinolinecarboxylic acid

CFLX : Ciprofloxacin

CI-934 : 1-Ethyl-7-(3-ethylaminomethyl-1-pyrrolidinyl)-6,8-difluoro-1,4-dihydro-4-oxo-3-quinolinecarboxylic acid

Table 2 In vitro antibacterial activity (clinical isolates)

| Clinical isolates : No. of strains | MIC (μg/ml) | | | | |
|---|---|---|---|---|---|
| | Exp.25 | Ref.2 | CFLX | OFLX | NFLX |
| S. aureus :21 | 0.05 | 0.10 | 0.39 | 0.39 | 1.56 |
| MR-S. aureus :46 | 0.10 | 0.39 | 3.13 | 1.56 | 25 |
| S. epidermidis :10 | 0.05 | 0.10 | 0.20 | 0.39 | 0.78 |
| S. pyogenes : 6 | 0.10 | 0.39 | 0.39 | 1.56 | 1.56 |
| S. pneumoniae : 6 | 0.10 | 0.78 | 1.56 | 3.13 | 12.5 |
| E. faecalis : 5 | 0.20 | 0.78 | 0.78 | 3.13 | 6.25 |
| N. gonorrhoeae :16 | 0.013 | 0.006 | 0.025 | 0.05 | 0.10 |
| N. meningitidis : 3 | 0.006 | 0.006 | 0.006 | 0.025 | 0.05 |
| B. catarrhalis :21 | 0.05 | 0.05 | 0.10 | 0.20 | 1.56 |
| H. influenzae :26 | 0.013 | 0.025 | 0.05 | 0.10 | 0.20 |
| E. coli :25 | 0.013 | 0.025 | 0.025 | 0.10 | 0.10 |
| P. pneumoniae :25 | 0.05 | 0.20 | 0.20 | 0.78 | 0.78 |
| E. cloacae :21 | 0.013 | 0.05 | 0.05 | 0.10 | 0.10 |
| S. marcescens :20 | 0.05 | 0.05 | 0.05 | 0.20 | 0.10 |
| P. mirabilis :10 | 0.05 | 0.05 | 0.05 | 0.20 | 0.10 |
| P. vulgaris : 6 | 0.05 | 0.05 | 0.05 | 0.10 | 0.05 |
| M. morganii : 8 | 0.025 | 0.10 | 0.05 | 0.10 | 0.05 |
| C. freundii : 9 | 0.10 | 0.20 | 0.10 | 0.39 | 0.39 |
| P. aeruginosa :27 | 0.20 | 1.56 | 0.39 | 3.13 | 1.56 |
| P. maltophilia : 9 | 0.78 | 1.56 | 12.5 | 6.25 | 25 |
| Acinetobacter sp. :13 | 0.10 | 0.78 | 1.56 | 3.13 | 12.5 |
| Alcaligenes sp. : 9 | 0.78 | 6.25 | 6.25 | 6.25 | 100 |
| Campylobacter jejuni :20 | 0.05 | 0.20 | 0.20 | 0.39 | 0.78 |
| Bacteroides sp. :17 | 0.10 | 0.39 | 6.25 | 3.13 | 50 |

OFLX : Ofloxacin

NFLX : Norfloxacin

Experiment 2. Frequency of spontaneous resistant mutants

Each test strain was grown overnight in Mueller-Hinton broth (Difco) at 37 °C, and then 0.1 ml of each culture broth was inoculated into 10 ml of Mueller-Hinton broth. After incubation at 37 °C for 6 hours by shaking, the bacterial cells were harvested by centrifugation at 3,000 rpm for 15 minutes. The pellets were suspended in 5 ml of fresh Mueller-Hinton broth. One portion (0.1 ml) of these bacterial suspensions was inoculated onto Muller-Hinton agar containing each compound at 2, 4 or 8 fold MIC, and then plates were incubated at 37 °C for 48 hours. Table 3 shows the frequency of resistant mutants to the present compound or to the reference compounds. The mutation frequency was calculated as follows: the number of colonies appeared on drug-containing agar plate/total number of the viable cells of inoculation. Spontaneous mutation frequency of present compound was significantly lower than those of the reference compounds.

Table 3 Frequency of spontaneous resistant mutants

| Strains | Conc. of Drugs | Example 25 | Ref.2 | CFLX |
|---|---|---|---|---|
| S. aureus 209 P | 2MIC | $2.9 \times 10^{-8}$ | - | $3.6 \times 10^{-7}$ |
| | 4MIC | $<2.9 \times 10^{-9}$ | $5.1 \times 10^{-8}$ | $1.3 \times 10^{-7}$ |
| S. aureus Smith | 2MIC | $<2.9 \times 10^{-9}$ | $3.6 \times 10^{-7}$ | $4.1 \times 10^{-8}$ |
| E. cloacae IID 977 | 2MIC | $6.7 \times 10^{-10}$ | $3.1 \times 10^{-7}$ | $1.0 \times 10^{-8}$ |
| | 4MIC | $<6.7 \times 10^{-10}$ | $<6.7 \times 10^{-10}$ | $<6.7 \times 10^{-10}$ |
| S. marcescens IID 618 | 2MIC | $1.5 \times 10^{-8}$ | $1.0 \times 10^{-7}$ | $4.1 \times 10^{-9}$ |
| | 4MIC | $<1.0 \times 10^{-9}$ | $<1.0 \times 10^{-9}$ | $<1.0 \times 10^{-9}$ |
| S. marcescens GN 7577 | 2MIC | $2.3 \times 10^{-7}$ | - | $1.2 \times 10^{-7}$ |
| | 4MIC | $<5.3 \times 10^{-10}$ | $9.9 \times 10^{-7}$ | $<5.3 \times 10^{-10}$ |
| | 8MIC | $<5.3 \times 10^{-10}$ | $2.1 \times 10^{-9}$ | $<5.3 \times 10^{-10}$ |
| P. aeruginosa IFO 12689 | 2MIC | $4.5 \times 10^{-8}$ | $5.5 \times 10^{-7}$ | $1.5 \times 10^{-7}$ |
| | 4MIC | $<6.3 \times 10^{-10}$ | $<6.3 \times 10^{-10}$ | $1.9 \times 10^{-9}$ |
| P. aeruginosa IFO 1210 | 2MIC | $<6.7 \times 10^{-10}$ | $7.7 \times 10^{-8}$ | - |
| | 4MIC | $<6.7 \times 10^{-10}$ | $<6.7 \times 10^{-10}$ | $7.0 \times 10^{-8}$ |

Experiment 3. In vivo antibacterial activity against systemic infections in mice

The infections were produced by intraperitoneal injection of suspension of the bacterial cells. The compounds were administered orally at an hour after the injection. Five male ICR mice were used for each dosage. The 50 % effective dose ($ED_{50}$) which protects 50 % of animals from infectious death was determined from the relation between dosage and survival rate. The efficacy of the present compound are shown in Table 4 in comparison with those of the reference compounds. The present compound was more effective than the reference compounds against all bacterial infections tested. Particularly, it showed excellent efficacy against S. pneumoniae infection, on which the reference compounds were ineffective.

Table 4 In vivo antibacterial activity against systemic infections in mice

| Compound | $ED_{50}$ | | | |
|---|---|---|---|---|
| | E. coli ML 4707 | P. aeruginosa IID 1210 | S. aureus Smith | S. pneumoniae S-4288 |
| Example 25 | 0.9 | 2.9 | 2.0 | 13.0 |
| CFLX | 1.5 | 4.5 | 31 | >100 |
| OFLX | 2.1 | 12.1 | 17 | >100 |
| NFLX | 8.9 | 18.9 | - | >100 |

Experiment 4. Therapeutic effect in an experimental local infection with anaerobic bacteria in granuloma pouches in rats

Under ether anesthesia, male Wistar rats (7 weeks old) were injected subcutaneously on the back with 20 ml of germ-free air (sterilized by membrane filtration, 0.45 μm pore size) by means of a syringe. Continuously, an inflammatory reaction was induced by injecting one ml of sterile croton oil solution (1 % in olive oil) into the formed air pouch. After 2 days, the air was removed. On the eighth day, 0.5 ml of Bacteroides fragilis 2 culture ($10^6$ CFU/ml) were injected into bore granuloma pouches. Each compound was administered orally or subcutaneously at 2 hours after infection. Three animals were used for each drug group per experiment and 0, 6 and 24 hours after the administration of the compounds, samples of exudate were taken from the pouches to determine the number of viable bacteria. During experiment, intake of food and water was free. The results are shown in Table 5. It was known that the activity of the existing quinolonecarboxylic acid compounds against obligate anaerobic bacteria was very weak. However, the present compound showed much stronger antibacterial activity against anaerobic bacteria not only *in vitro* but also *in vivo* than the other quinolonecarboxylic acids. This property is also one of excellent characters of the present comound. As shown in Table 5, this experiment shows that the present compound is more effective than cefoxitin (CFX), clindamycin (CLDM) and metronidazole (MND) usually used for treatment of anaerobic infections clinically.

Table 5 Therapeutic effect in an experimental local infection in granuloma pouches in rats

| Drugs | | Viable cells count (log) / ml | | |
|---|---|---|---|---|
| | | 0 hr | 6 hr | 24 hr |
| Control | | $5.1 \pm 0.064$ | $6.2 \pm 0.10$ | $9.6 \pm 0.10$ |
| Example 25 | 25 mg/kg, p.o. | | $4.6 \pm 0.31$* | $4.5 \pm 0.15$** |
| CFX | 50 mg/kg, s.c. | | $3.9 \pm 0.25$** | $6.3 \pm 0.31$** |
| CLDM | 25 mg/kg, s.c. | | $4.6 \pm 0.058$** | $6.4 \pm 0.38$** |
| MND | 50 mg/kg, p.o. | | $5.2 \pm 0.10$** | $5.7 \pm 0.60$** |
| Control | | $5.6 \pm 0$ | $6.2 \pm 0.31$ | $9.3 \pm 0.17$ |
| Ref.2 | 25 mg/kg, p.o. | | $5.1 \pm 0.21$ | $4.5 \pm 0.55$ |

Mean ± S.D. (n=3).

*, **:significantly different from control by paired T-test ($P < 0.05$, $P < 0.01$, respectively).

Experiment 5. Acute toxicity in mouse

Acute toxicity of the present compound was investigated in male mice and each drug was administered intravenously. $LD_{50}$ value of the present compound is almost same as that of ciprofloxacin.

Table 6 Acute toxicity in mouse

| Compound | $LD_{50}$ (mg/kg) |
|---|---|
| Example 25 | 242 (221-279) |
| CFLX | 231 (212-255) |

(i.v.)

Experiment 6. Subacute toxicity in dog

Subacute toxicity study of the present compound was carried out in beagle dogs (10 weeks old, n=3). To animals 20 mg/kg/day of the present compound for 8 days and after that 50 mg/kg/day for 7 days was administered orally.

No remarkable changes in body weight and general conditions, and no abnormality with regard to hematological examination and serum biochemical analysis were observed.

Experiment 7. Absorption, excretion and metabolism in animals

1) Rats

The present compound and ciprofloxacin were administered orally to rats, at the dose of 10 mg/kg, respectively, and then bloods were sampled at indicated time after administration. The

drug concentrations in serum were measured by bioassay. Urinary and biliary excretion within 24 hours after administration were also determined by bioassay. The results are shown in Fig. 1 and Table 7. Serum levels of present compound were three times higher than that of ciprofloxacin. Similarly, urinary and biliary recovery of present compound were also 2.4- and 7 times higher than that of ciprofloxacin, respectively.

Table 7 Urinary and biliary excretion

(Rats, 10 mg/kg, p.o.)

| Compound | Excretion rate (% of dose) | |
|---|---|---|
| | Urine | Bile |
| Example 25 | 21.7 | 13.4 |
| CFLX | 8.9 | 1.9 |

0-24 hours

2) Dogs

A 2 mg dose of the present compound was administered to beagle dogs orally or intravenously, and the serum levels were measured by HPLC (High-performance liquid chromatography). The results are shown in Fig. 2. Serum levels of the present compound by oral route were almost equal to those by intravenous route, suggesting the excellent oral absorption of the present compound. Study of urinary metabolites indicates that the present compound may be hardly metabolized.

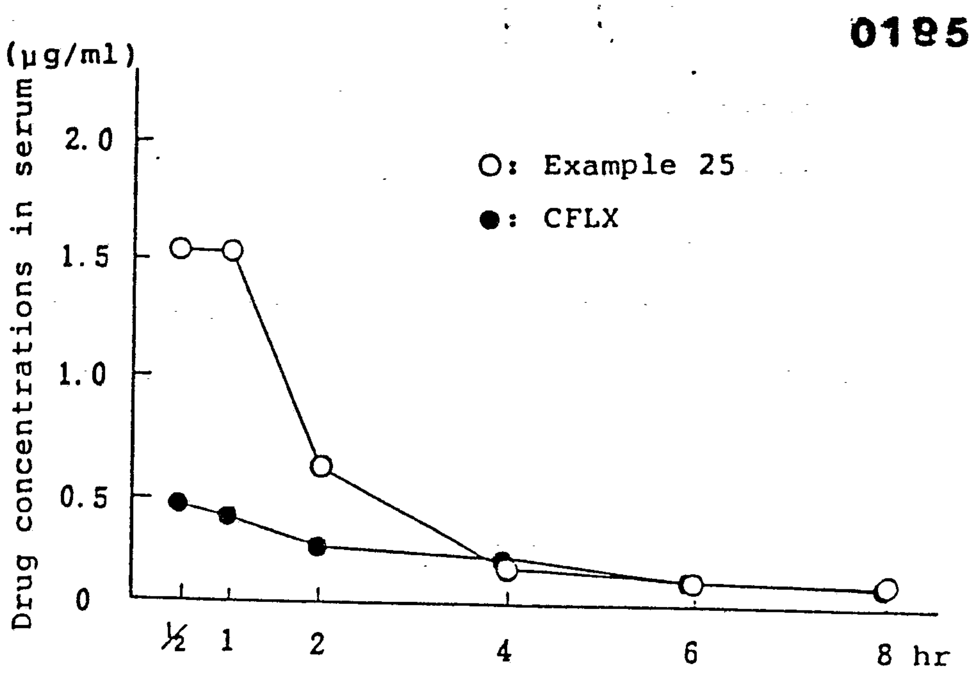


Fig. 1 Drug concentrations in serum
(Rat, 10 mg/kg, p.o.)

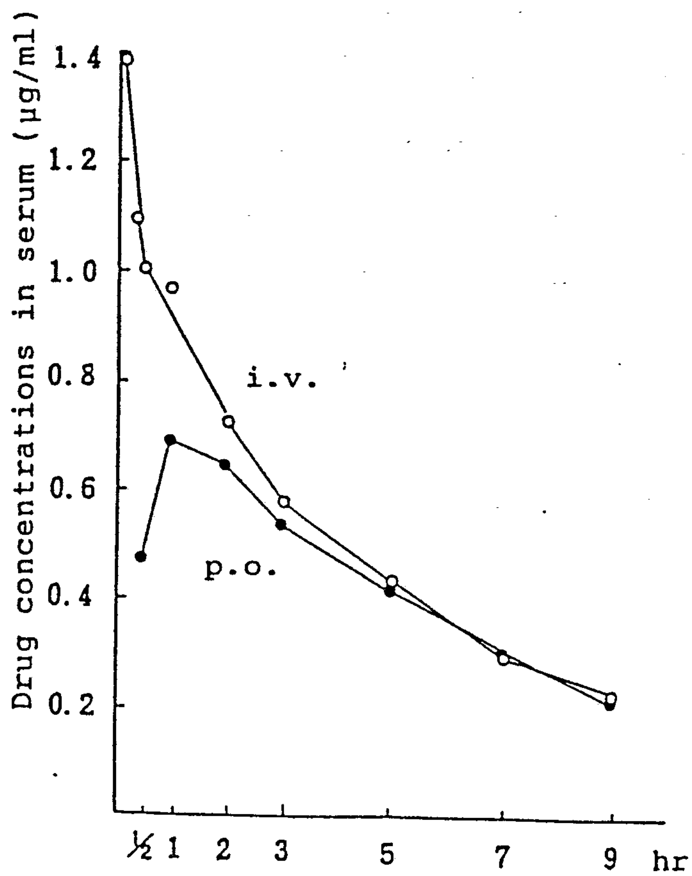


Fig. 2 Drug concentrations in serum
(Dog, 2 mg/kg)

Experiment 8. Mutagenicity assay

As for antibacterial agents, assessment of mutagenicity using bacteria is somewhat difficult because of their activity on the test bacteria. Then, in order to assess mutagenic activities at higher concentration, we selected a strain which showed resistant to test agents and also retained the properties of the parent test strain. Using this resistant test strain, it was possible to assess at 10-fold higher concentration compared with the parent strain.

When reverse mutation assay using nalidixic acid-resistant Salmonella typhimurium TA98 strain ($nal^r$) was done with the present compound and ciprofloxacin, within measurable concentration, with and without the drug metabolic activation, ciprofloxacin induced 2-fold $His^+$ revertant colonies compared with the solvent control, but the present compound exhibit no increment of $His^+$ revertant colonies compared with the control (Table 8). These results suggested that ciprofloxacin might have a weak mutagenecity.

Table 8 Mutagenic activity in S. typhimurium

| μg/plate | S9-mix | TA98 ($nal^r$) Example 25 | TA98 ($nal^r$) CFLX |
|---|---|---|---|
| | | His$^+$ revertants per plate | |
| Control | - | 27 | 27 |
| 0.001 | - | | |
| 0.005 | - | 28 | |
| 0.01 | - | 29 | |
| 0.05 | - | 26 | 28 |
| 0.1 | - | 16* | 44 |
| 0.5 | - | 0* | 47 |
| 1.0 | - | | 55 |
| 5.0 | - | | 0* |
| 0.2 (4NQQ[1]) | - | 190 | 190 |
| Control | + | 49 | 49 |
| 0.001 | + | | |
| 0.005 | + | 60 | |
| 0.01 | + | 48 | |
| 0.05 | + | 39* | 47 |
| 0.1 | + | 6* | 76 |
| 0.5 | + | 0* | 103 |
| 1.0 | + | | 30* |
| 5.0 | + | | 0* |
| 0.5 (2AA[2]) | + | 277 | 277 |

* : Background growth was inhibited

1): 4-Nitroquinoline 1-oxide

2): 2-Aminoanthracene

What is claimed is:

1. A compound of the formula (I);

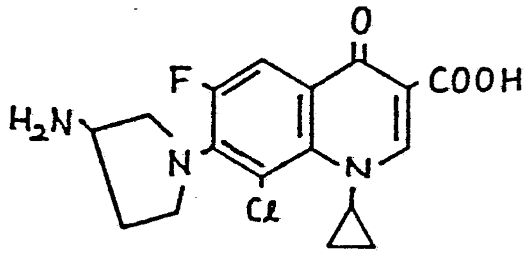

the hydrates or the pharmaceutically acceptable acid addition or alkali salts thereof.

2. A process for the preparation of a compound of the formula (I),

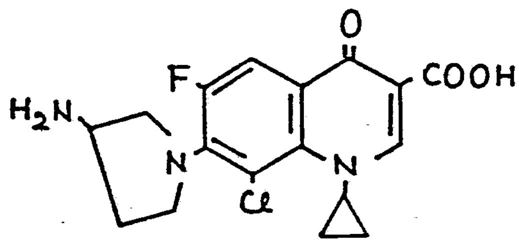

which comprises condensing a compound of the formula (II);

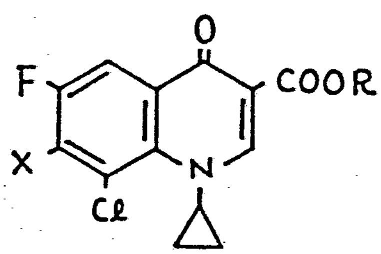

[wherein R is a hydrogen atom or a lower alkyl group, X is a halogen atom], with a compound of the formula (III);

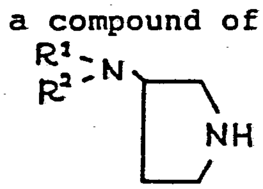

[wherein $R^1$ and $R^2$ are each independently a hydrogen atom or a protecting group of an amino group, or are a protecting group of

an amino group which work together]; however in which, when both or either of $R^1$ and $R^2$ are a protecting group of an amino group, the condensed product is followed by removal of the protecting group to an amino derivative and when R is a lower alkyl group, the condensed product is followed by saponification to the carboxylic acid derivative.

3. An antibacterial pharmaceutical composition containing the compound according to claim 1 and an inert pharmaceutical acceptable carrier.

0195316

European Patent Office

# EUROPEAN SEARCH REPORT

Application number

EP 86 10 2938

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP-A-0 106 489 (WARNER-LAMBERT) * Claims 1,18 * | 1,3 | C 07 D 401/04<br>A 61 K 31/47 |
| Y | * Example 15 * | | |
| Y | GB-A-2 057 440 (KYORIN SEIYAKU KABUSHIKI) * Claims 1,15; examples 5,6 * | 1,3 | |
| Y | BE-A- 899 399 (KYORIN SEIYAKU KABUSHIKI) * Claims 1,3 * | 1,3 | |
| P,X | EP-A-0 153 163 (WARNER LAMBERT) * Page 3, lines 15-19; pages 4,5; page 6, lines 1-4; page 12, lines 1-5 * | 1,3 | TECHNICAL FIELDS SEARCHED (Int. Cl 4)<br>C 07 D 401/00<br>A 61 K 31/00 |

The present search report has been drawn up for all claims

| Place of search | Date of completion of the search | Examiner |
|---|---|---|
| THE HAGUE | 25-06-1986 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document